# EUROPEAN PATENT SPECIFICATION

(11) **EP 3 603 720 B1**
(45) Date of publication and mention of the grant of the patent: **09.03.2022**
(21) Application number: 18186489.3
(22) Date of filing: 31.07.2018
(51) Int. Cl.: A61M 25/00

(54) **CATHETER DESOBSTRUCTION APPARATUS AND SYSTEM**
KATHETERDESOBSTRUKTIONSANORDNUNG UND SYSTEM
OUTIL DE DÉSOBSTRUCTION DE CATHÉTER ET SYSTÈME

(43) Date of publication of application: 05.02.2020
(73) Proprietor: CaDo Medial Solutions GmbH, 91058 Erlangen (DE)
(72) Inventor: Elsässer, David, 91052 Erlangen (DE)
(74) Representative: Wittmann, Günther

(56) References cited:
- WO-A1-2017/079643
- US-A- 4 698 058
- US-A- 5 030 213
- US-A1- 2009 264 833

## Description

The present invention relates to a catheter desobstruction apparatus, a catheter desobstruction drive and a use therefore, particularly for desobstructing urine catheters.

### Field of the invention

Catheters for draining the bladder of a human are known since ancient times. Catheters are used, if a patient cannot drain his bladder. Catheters can be inserted transurethral or suprapubic.

A frequent problem in use of catheters is that the lumen of the catheter may get obstructed, such as by salt. Generally, such obstructions occur in the proximal portion of the catheter close to the inlet opening of the catheter.

If a permanent catheter gets obstructed, the patient will feel strong pain requiring acute intervention. In the prior art the catheter may be flushed back, e.g. by a syringe. In many cases the obstruction cannot be eliminated by flushing. Consequently, the catheter has to be changed under emergency conditions by a physician.

If the catheter is not changed as early as possible under emergency conditions, additional complications may develop by the patient, such as sepsis, kidney failure, inflammation of the urethra or the like.

Currently no catheter desobstruction apparatus for the desobstructing of an inserted catheter is commercially available.

### Prior art

WO 87/02255 discloses an ultrasonic self-cleaning catheter system.

US 5,030,213 discloses a catheter router assembly for cleaning salt blockages in a previously inserted catheter. A length of flexible, stainless steel cable is provided and a silver solder tip is applied at the proximal end of the cable. The silver solder tip is then machined to form a suitable cutting edge for cutting or drilling through the body salts which clog the catheter.

US 2005/0267421 A1 discloses a catheter cleaner comprising a head, a rod and a handle. The head defines a tip and cutting edges. The tip may have a curved configuration which may be operative to assist the insertion of the cleaner into an indwelling catheter.

US 6,183,450 B1 discloses a catheter cleaning device for cleaning a catheter of obstructions. The catheter cleaning device comprises an elongate hollow housing, a guide member encircling the housing and including a bar extending across a diameter thereof and through first and second slots of the elongate hollow housing. A guide wire extends from the bar.

US 2009/0264833 A1 discloses a device for clearing obstructions from a medical tube, such as a chest tube. The device features a guide wire that extends from a drainage canister and can be advanced and withdrawn through a medical tube, such as a chest tube wire and actuator. The guide wire is actuated so as to maintain the sterile field within the chest tube and associated suction partway.

### Summary of the invention

It is an object of the present invention to provide a catheter desobstruction apparatus for desobstructing a catheter inserted into a patient.

The object of the present invention is solved by a catheter desobstruction apparatus according to claim 1.

The depending claims claim preferred embodiments.

The invention discloses a catheter desobstruction apparatus comprising a flexible catheter cleaning cable comprising at its proximal end a desintegration tool and at its distal end a cable coupling. The flexibel catheter cleaning cable is adapted to be introduced into a lumen of a catheter. The catheter desobstruction apparatus further comprises a drain element having a first opening, a second opening and a third opening. The first opening is adapted to be connected to the catheter. The first and second opening are adapted that the catheter cleaning cable can pass through the first and second opening. The third opening is in fluid communication with the first opening.

The flexible catheter cleaning cable can be introduced into the lumen of the catheter for removing the obstruction in the lumen of the catheter. As soon as the obstruction has been removed, urine may flow from the bladder to the drain element. The drain element directs the urine to the third opening to which a tube, a pipe, a bag and/or a container may be connected.

The disintegration tool can be a blade, a drill, a crushing tool or the like.

At the first, the second and third opening a Luer cone may be arranged.

The present invention has the advantage that urine released from the bladder into the catheter flows in a determined way to a vessel without contaminating the bed of the patient, the operating table or the like.

The first opening is arranged opposite to the second opening. In this embodiment the flexible catheter cleaning cable passes in a straight manner through the drain element.

The drain element may have a generally T-shaped configuration. The first opening and the second opening may be arranged opposite to each other. The third opening may be arranged between the first and second opening and/or perpendicular to the first and second opening.

The flexible catheter cleaning cable passes through the first and second opening.

The drain element and the flexible catheter cleaning cable may be located in a sterile packing. The flexible catheter cleaning cable passes through the first and second opening, such that the catheter desobstruction apparatus is ready for use after opening the sterile packing.

The catheter desobstruction apparatus may further comprise a sealing arranged in use around the flexible catheter cleaning cable blocking flow of a liquid (e.g. urine) from the first opening to the second opening.

In one embodiment the drain element comprises a first conduit extending from the first opening to the second opening and a second conduit branching off from the second opening, wherein the sealing is arranged between the second opening and the branch off of the conduit. The sealing does not have to extend from the second opening to the branch off of the second conduit. Thereby, release of urine by the second opening can be prevented. The urine will be released by the drain element only by the third opening.

The invention also discloses a catheter desobstruction drive having a drive coupling adapted to be connected with a cable coupling of a flexible catheter cleaning cable. The catheter desobstruction drive comprises a motor rotatory driving the drive coupling. The drive coupling may rotate about a rotation axis passing through the center of the drive coupling. The catheter desobstruction drive may further comprise an actor translationally moving the drive coupling. The drive coupling may be moved along the rotation axis of the drive coupling. The catheter desobstruction drive may further comprise a force sensor adapted to determine the force acting on the drive coupling in the translational direction. The catheter desobstruction drive further comprises a controller adapted to control the motor and actor.

The catheter desobstruction drive may comprise an opening through which the flexible catheter cleaning cable can pass and a sealing adjacent to the opening through which the flexible catheter cleaning cable can pass and preventing that a liquid can enter the drive. The sealing may comprise bellows through which the catheter cleaning cable passes.

In one embodiment, the controller is adapted to instruct the motor to rotate the drive coupling and the actor to move the drive coupling in the proximal direction. Thereby, the obstruction is removed by the rotating desintegration tool at the distal end of the flexible catheter cleaning cable around its longitudinal axis.

In another embodiment, the controller is adapted to instruct the actor to move the drive coupling into the proximal direction and to instruct the motor not to rotate the drive coupling. If a force detected by the force sensor exceeds a predetermined threshold, the controller instructs the actor to move the drive coupling in the distal direction for a first predetermined distance. If the force detected by the force sensor exceeds a predetermined threshold, the desintegration tool of the flexible catheter cleaning cable might have contacted the obstruction. Thereafter, the desintegration tool is removed from the obstruction by moving the flexible cleaning cable from the obstruction, when the catheter desobstruction drive is connected to the above described catheter desobstruction apparatus. When the actor has moved the drive coupling the first predetermined distance in the distal direction, the controller instructs the motor to rotate the drive coupling and the actor to move the drive coupling in the proximal direction. Thereby, the obstruction is removed by the rotating desintegration tool at the distal end of the flexible catheter cleaning cable around its longitudinal axis. In this embodiment the disintegration tool only rotates in the portion close to the obstruction. Thereby, damaging of the catheter may be avoided.

The controller may be further adapted to instruct the actor to move the drive coupling a predetermined distance in the proximal direction and the motor to rotate the drive coupling. Thereby, the obstructions in the lumen of catheter are removed by the rotating desintegration tool at the proximal end of the flexible catheter cleaning cable. If the actor has moved the drive coupling the predetermined distance in the proximal direction, the controller instructs the actor to move the drive coupling a second predetermined distance in the distal direction and the motor to rotate the drive coupling. The flexible catheter cleaning cable is moved partially outward of the catheter and the desintegration tool at the distal end of the flexible catheter cleaning cable passes again the location which was obstructed. By the rotating desintegration tool residues of the obstruction are removed. Since the flow of urine is not blocked after removing the obstruction the urine is released from the bladder and flows through the catheter to a container connected to the third opening. Thereby, any residues of the obstruction in the lumen of the catheter are flushed.

The predetermined distance may be a set distance. The predetermined distance may be computed dynamically, e.g. based on a force exerted on the actor and/or motor. The force exerted on the actor and/or motor may fall under a predetermined threshold, after the obstruction is removed by the disintegration tool.

The actor may be an electric actor and the force sensor may be a current sensor measuring the current supplied to the electric actor.

The invention also discloses a catheter desobstruction system comprising the catheter desobstruction apparatus described above and the catheter desobstruction drive described above.

Disclosed but not forming part of the invention is the use of the catheter obstructuon system for desobstructing a catheter. The proximal end of the flexible catheter cleaning cable is introduced into a lumen of the catheter. This step may be performed by nursing staff. Thereafter, the drive coupling of the catheter desobstruction drive is coupled with cable coupling of the flexible catheter cleaning cable of the catheter desobstruction apparatus, e.g. by the nursing staff. Then, the catheter desobstructing method is activated by pressing a button on the desobstruction drive. The controller of the catheter desobstruction device controls the catheter desobstruction method.

In one example, the proximal end of the flexible catheter cleaning cable is then rotated by the motor of the catheter desobstruction drive and the flexible catheter cleaning cable is moved in the proximal direction by the actor. Thereby, the obstruction in the lumen of the catheter is removed.

According to another example, the proximal end of the flexible catheter cleaning cable is moved into the proximal direction by the actor of the catheter desobstruction drive without rotating the distal end. If a force detected by the force sensor of the catheter desobstruction drive exceeds a predetermined threshold, the flexible catheter cleaning cable is moved in the distal direction for a first predetermined distance by the actor. When the flexible catheter cleaning cable has been moved the first predetermined distance in the distal direction, the proximal end of the flexible catheter cleaning cable is rotated by the motor of the catheter desobstruction drive and the flexible catheter cleaning cable is moved in the proximal direction by the actor. Thereby, the obstruction in the lumen of the catheter is removed.

If the flexible catheter cleaning cable has been moved the predetermined distance in the proximal direction, the flexible catheter cleaning cable is moved a second predetermined distance in the distal direction by the actor and the flexible catheter cleaning cable is rotated by the motor. Thereby, residues of the removed obstruction are removed. Further, the residues of the obstruction are flushed by the urine released from the bladder to the third opening of the drain element and a device connected thereto. If the flexible catheter cleaning cable has been moved the second predetermined distance in the distal direction, moving of the flexible catheter cleaning cable is stopped and rotating of the proximal end of the flexible catheter cleaning cable is stopped.

The predetermined distance may be a set distance. The predetermined distance may be computed dynamically, e.g. based on a force exerted on the actor and/or motor. The force exerted on the actor and/or motor may fall under a predetermined threshold, after the obstruction is removed by the disintegration tool.

Thereafter, a service staff can remove the flexible catheter cleaning cable from the lumen of the catheter.

The present invention provides the advantage that an obstruction in an inserted catheter can be removed by nursing staff and no supervision by a physician is necessary. Since the inventive apparatus may be operated by nursing staff, the obstruction in the catheter can be removed within a short period after the obstruction occurred. Thereby, comfort of the patient is increased and additional complications described above are avoided.

### Brief description of the drawings

The invention is now explained with reference to the drawings showing non-limiting embodiments of the invention, wherein
Figure 1 is a schematic view of a catheter desobstructing system;
Figure 2 show a catheter desobstructing apparatus in a sterile packing; and
Figure 3 shows a flowchart of the exemplary use (not claimed).

### Detailed description of the drawings

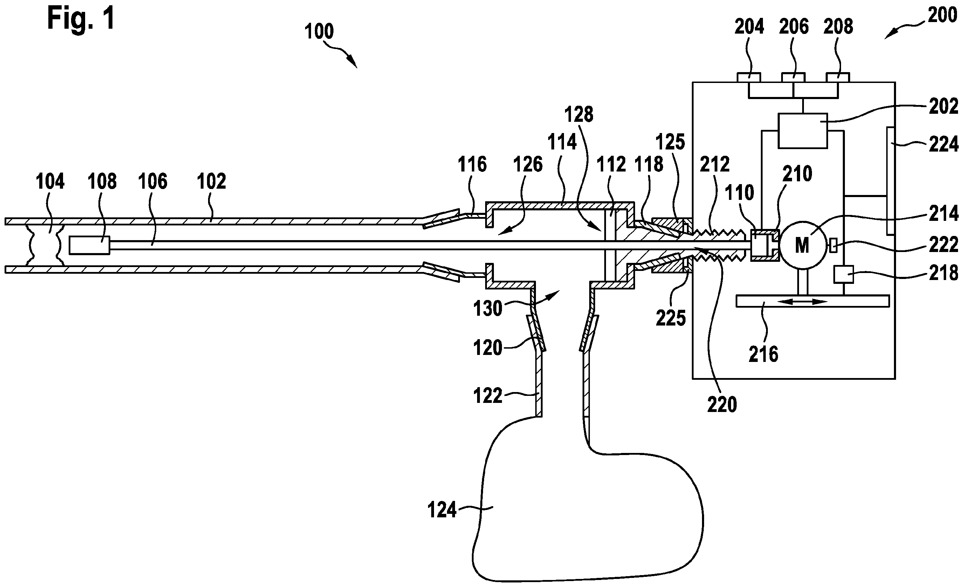

Figure 1 depicts a schematic view of the catheter desobstruction system comprising the catheter desobstruction apparatus 100 and the catheter desobstruction drive 200. The desobstruction apparatus 100 comprises a cable 106, i.e. a flexible catheter cleaning cable, wherein at the proximal end of the cable 106 a blade 108 is arranged. The blade 108 is an example of a desintegration tool and alternatively to the blade a crushing tool, a drill or the like may be used.

The proximal end of the catheter 102 is positioned releasably in the bladder of the patient such that the patient can release urine. The catheter 102 has been inserted by a physician, if a patient cannot release urine.

In use, if a catheter is obstructed, the cable 106 and the blade 108 are inserted into the lumen of a catheter 102 by a user, such as nursing staff. The catheter 102 is obstructed by salt 104 such that urine cannot flow from the bladder of a patient through the catheter 102.

The distal end portion of the catheter 102 is pushed by the user on a first cone 116 adjacent to a first opening 126 of a drain element 114. The drain element 114 comprises the first cone 116 adjacent to the first opening 126, a second cone 118 adjacent to a second opening 128 and a third cone 120 adjacent to a third opening 130. A tube 122 connected to a bag 124 is connected to the third cone 120. Urine can flow from the catheter 102 through the first cone 116, the first opening 126, the third opening 130 and the third cone 120 of the drain element 114 to the tube 122 and the bag 124.

In one embodiment the first cone 116, the second cone 118 and/or the third cone 120 may be a Luer cone.

The cable 106 extends through the first cone 116, the first opening 126, the second opening 128 and the second cone 118 of the drain element 114. The first cone 116 and the second cone 118 are arranged opposite to each other. The third cone 120 is arranged essentially perpendicular to the first cone 116 and the second cone 118. The drain element 114 is generally T-shaped. In a passage between the third cone 120 and the second cone a sealing 112 is arranged around the cable 106 for avoiding that a fluid can flow to the third cone 118.

To the second cone 118 a connector 125 for connecting the drain element 114 to a drive 200 explained below is arranged.

The drive 200 is adapted to rotate the cable 106 around its longitudinal axis and to move the cable along its longitudinal axis. The drive 200 comprises a motor 214 for rotating the cable around its longitudinal axis and a carriage 216 for displacing the cable 106 along its longitudinal axis. The motor 214 is connected to a controller 202 by the motor controller 222, and the carriage 216 is connected via a carriage controller 218 to the controller 202.

The controller is connected to a power-on switch 204, a start switch 206 and a release switch 208. If a user presses on the power-on switch 204, the drive is supplied with power and activated. As soon as the user presses the start button 206 an automatic method for desobstructing the catheter 102 is started as described below in detail.

The drive 200 further comprises a complimentary connector 225 for receiving the connector 125 of the drain element 114. The connector 125 of the drain element 114 and the complimentary connector 225 of the drive define the positional relationship between the drive 200, the drain element 114 and the cable 106.

The cable 106 comprises at its distal end a cable coupling 110. In one embodiment the cable coupling 110 may have a square cross section. The cable coupling 110 of the cable 106 is received by a drive coupling 210. The drive coupling 210 may be a clamp, a clamp jug, a clamping nut or the like that may be operated by the controller 202.

The drive further comprises bellows 212 or a suitable seal arranged at the opening 220 adjacent to the complimentary connector 225 for avoiding that a liquid can enter the drive, i.e. if the seal 112 fails. The cable 106 may pass in its longitudinal direction (expansion direction of the bellows) through the bellows. The bellows can be expanded or contracted, if the carriage 216 moves the motor 214 and the drive coupling 210 and the cable coupling 110 as well as the cable 106 connected thereto.

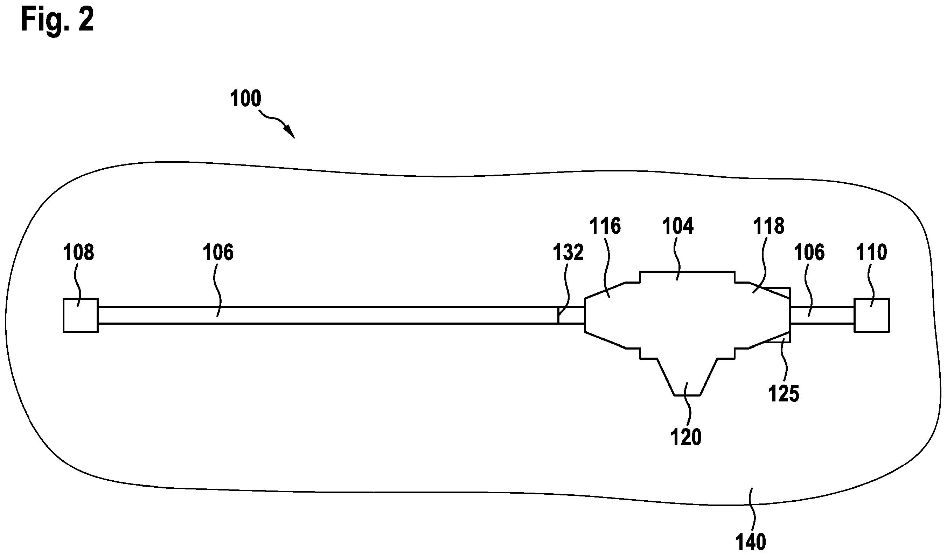

Figure 2 shows the catheter desobstruction apparatus 100, as it is delivered to a user, such as a nursing staff. The desobstruction apparatus 100 is accommodated in a sterile packing 140. The cable 106 runs from the first cone 116 to the second cone 118. The blade 108 is arranged at the proximal end of the cable 106 and the cable coupling 110 is arranged at the distal end of the cable 106. The connector 125 is arranged on the second cone 118.

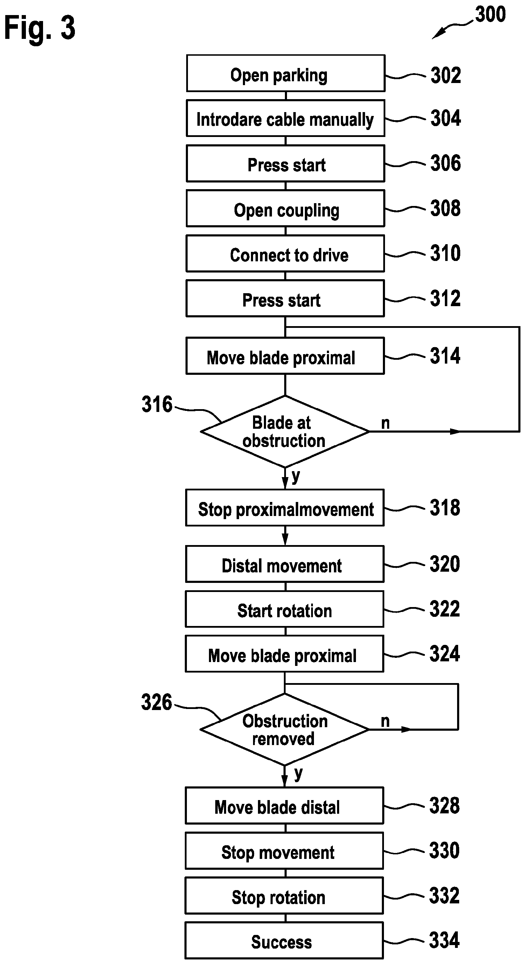

With reference to figure 3 a method 300 for using the catheter desobstruction system 100, 200 is described with respect to a urine catheter 102. In a first step 302 a user, such as nursing staff opens the sterile packing 140. Then, the user introduces the blade 108 and the cable 106 up to a predetermined position indicated by a marking 132 into the catheter 102 in step 304. The obstructions 104 in a urine catheter are generally formed by salt and are generally located close to the proximal opening of the catheter 102.

Then, in step 306 the user presses the start button 206 of the drive after having powered on the drive by pressing the power on button 204. The controller 202 instructs the drive coupling 210 to open in step 308. Thereafter, in step 310 the user connects the desobstruction apparatus 100 with the drive 200. Thereby, the cable connector 110 is positioned in the drive connector 210 and the connector 125 of the drain element 114 is releasably connected with the complimentary connector 225 of the drive 200.

In the following step 312 the user presses the start button again. The controller 202 instructs the drive coupling 210 to clamp the cable coupling 110 fixedly.

In step 314 the automatic desobstruction procedure commences. Particularly, in step 314 the controller 202 instructs the carriage 216 to move the blade 108 in the proximal direction. The carriage controller 218 monitors the current supplied to the carriage 216. If the current detected by the carriage controller 218 is below a predetermined threshold in step 316 the controller instructs the carriage 216 to move the blade 108 in the proximal direction. As soon as the carriage controller 218 detects in step 316 that the current supplied to the carriage 216 exceeds a predetermined threshold, the controller 202 stops the movement of the carriage 216 in step 318. The current supplied to the carriage 216 is increased, because the blade 108 touched the obstruction 104.

Then, the controller 202 proceeds with step 320 and instructs the carriage 216 to move the blade 108 a first predetermined distance in the distal direction.

As soon as the carriage 216 has moved the blade 108 the first predetermined distance in the distal direction, the controller 202 instructs the carriage 216 to stop movement in the distal direction. Thereafter, the controller 202 instructs in step 322 the motor 214 to rotate the blade 108 around the longitudinal axis of the cable106. The torque of the motor 214 is transferred by the drive coupling 210 to the cable coupling 110. In order to minimize friction of the cable 106 in the catheter 102, the cable 106 may be coated with Teflon or the like. In order to minimize friction, the cable may comprise cylindrical guides arranged along the cable 106. In another embodiment the cable 106 may be coated with silicone or plastics. Further, the controller 202 instructs the carriage 216 to move the blade 108 in the proximal direction in step 324.

As soon as the blade 108 touches the obstruction 104, the current supplied to the motors 214 will increase as detected by motor controller 222, due to the friction caused by the obstruction 104. Further, the current supplied to the carriage 216 increases as detected by the carriage controller 218 due to the friction caused by the obstruction 104. At least one of the current supplied to the motor 214 and the current supplied to the carriage 216 is monitored in step 326. As long as the current supplied to the motor 214 and/or the current supplied to the carriage 216 remain above a predetermined threshold, the obstruction 104 is removed by the blade and the blade 108 is continued to be rotated and moved in the proximal direction.

As soon as the controller 202 detects that the current supplied to the motor 214 and/or carriage 216 falls under the predetermined threshold, the obstruction 104 has been removed by the blade. The controller proceeds to step 328. Further, as soon as the obstruction 104 is removed, urine flows through the catheter and the first opening 126 and the third opening 130 of the drain element 114 into the bag 124.

In step 328 the controller 202 instructs the carriage 216 to move the blade 108 in the distal direction and the motor 214 to rotate the blade. Thereby, debris of the obstruction 104 can be removed from the catheter 102.

As soon as the carriage 216 has moved the blade 108 a second predetermined distance in the distal direction, the controller 202 instructs in step 330 the carriage 216 to stop the movement in the distal direction. Thereafter, in step 332 the controller 202 instructs the motor 214 to stop rotating.

The desobstruction method has been finalized successfully and a message indicating success of the desobstruction method is displayed on the display 224 of the drive 200. If a user presses the release switch 208, the controller instructs the drive coupling 210 to release the cable coupling 110 and the cable is disconnected from the cleaning device.

The user removes the cable 106 and the drain element 104 from the catheter 102 and removes the drive 200 from the drain element 114, after the controller has instructed the drive coupling 210 to release the cable coupling 110 after successfully finalizing the desobstruction method.

The drive 200 may be reused. The desobstruction apparatus 100 can be discarded or cleaned and sterilized for further usage.

As soon as the drain element 114 is removed from the catheter 102 the urine bag of the patient can be connected to the catheter.

The present invention provides the advantage that an acute obstruction of a urine catheter 102 can be desobstructed by nursing staff within a short time span. Thereby, pain of the patient is avoided, and additional complications such as sepsis, kidney failure, inflammation of the urethra or the like of the patient may be avoided. Further, costs can be reduced significantly since medical intervention by a physician or transportation of the patient to hospital can be avoided.

## Claims

1. A catheter desobstruction apparatus (100), comprising
- a flexible catheter cleaning cable (106) comprising at its proximal end a desintegration tool (108) and at its distal end a cable coupling (110), wherein the flexible catheter cleaning cable (106) is adapted to be introduced into a lumen of a catheter (30);
- a drain element (114) having a first opening (126), a second opening (128) and a third opening; wherein
- the first opening (126) is adapted to be connected to the catheter (30);
- the first and second opening (128) are adapted that the cleaning cable (106) can pass through the first opening (126) and second opening (128);
- the third opening (113) is in fluid communication with the first opening (116);
- the first opening (126) is opposite to the second opening (128); and
- the flexible catheter cleaning cable (106) passes through the first opening (126) and second opening (128).

2. The catheter desobstruction apparatus (100) according to claim 1, wherein the third opening (113) is adapted to at least one of the following:
- to be connected to a tube (122);
- to be connected to a pipe;
- to be connected to a bag (124);
- to be connected to a container.

3. The catheter desobstruction apparatus (100) according to claim 1 or 2, wherein the drain element (114) has a generally T-shaped configuration.

4. The catheter desobstruction apparatus (100) according to any one of claims 1 to 3, wherein the drain element (114) and the flexible catheter cleaning cable (106) are located in a sterile packaging (140).

5. The catheter desobstruction apparatus (100) according to any one of claims 1 to 4, further comprising a sealing (112) arranged in use around the flexible catheter cleaning cable (106) blocking flow of a liquid from the first opening (126) to the second opening (128).

6. The catheter desobstruction apparatus (100) according to claim 5, wherein the drain element (114) comprises a first conduit (116) extending from the first opening (126) to the second opening (128) and a second conduit (118) branching off from the second opening (128), wherein the sealing (112) is arranged between the second opening (128) and the branch off of the second conduit (118).

7. A catheter desobstruction system comprising the catheter desobstruction apparatus (100) according to any one of claims 1 to 6 and a catheter desobstructing drive (200), wherein
the catheter desobstruction drive (200), comprises
- drive coupling (210) adapted to be connected with the cable coupling (110) of the flexible catheter cleaning cable (106);
- a motor (214) rotatory driving the drive coupling (210);
- an actor (216) translationally moving the drive coupling (210);
- a force sensor (222) adapted to determine the force acting on the drive coupling (210) in the translational direction; and
- a controller (202) adapted to control the motor (214) and actor (216);
- wherein the controller (202) is adapted to the following:
- instructing the motor (214) to rotate the drive coupling (210) and the actor (216) to move the drive coupling (210) in the proximal direction.

8. The catheter desobstruction system of claim 7, further comprising:
- an opening (220) through which the flexible catheter cleaning cable (106) can pass; and
- a sealing (212) adjacent to the opening through which the flexible catheter cleaning cable (106) can pass and preventing that a liquid can enter the catheter desobstruction drive (200).

9. The catheter desobstruction system (200) of claim 7 or 8, wherein the controller (202) is further adapted to the following:
- instructing the actor (216) to move the drive coupling (210) a predetermined distance in the proximal direction and the motor (214) to rotate the drive coupling (210);
- if the actor (216) has moved the drive coupling (210) the predetermined distance in the proximal direction, instructing the actor (216) to move the drive coupling (210) a predetermined distance in the distal direction and the motor (214) to rotate the drive coupling (210); and
- if the actor (216) has moved the drive coupling (210) the second predetermined distance in the distal direction, instructing the actor (216) to stop moving the drive coupling (210) and the motor (214) to stop rotating the drive coupling (210).

10. The catheter desobstruction system (200) of claim 9, wherein the actor (216) is an electric actor and the force sensor (222) is a current sensor measuring the current supplied to the electric actor.

## Patentansprüche

1. Katheter-Desobstruktionsvorrichtung (100), umfassend
- ein flexibles Katheterreinigungskabel (106), das an seinem proximalen Ende ein Desintegrationswerkzeug (108) und an seinem distalen Ende eine Kabelkupplung (110) aufweist, wobei das flexible Katheterreinigungskabel (106) dazu ausgebildet ist, in ein Lumen eines Katheters (30) eingeführt zu werden;
- ein Ablaufelement (114) mit einer ersten Öffnung (126), einer zweiten Öffnung (128) und einer dritten Öffnung; wobei
- die erste Öffnung (126) dazu ausgebildet ist, mit dem Katheter (30) verbunden zu werden;
- die erste und die zweite Öffnung (128) so ausgebildet sind, dass das Reinigungskabel (106) durch die erste Öffnung (126) und die zweite Öffnung (128) geführt werden kann;
- die dritte Öffnung (113) in Fluidverbindung mit der ersten Öffnung (116) steht;
- die erste Öffnung (126) der zweiten Öffnung (128) gegenüber liegt; und
- das flexible Katheterreinigungskabel (106) durch die erste Öffnung (126) und die zweite Öffnung (128) verläuft.

2. Katheter-Desobstruktionsvorrichtung (100) nach Anspruch 1, wobei die dritte Öffnung (113) zu zumindest einem des Folgenden ausgebildet ist:
- um mit einem Schlauch (122) verbunden zu werden;
- mit einem Rohr verbunden zu werden;
- mit einem Beutel (124) verbunden zu werden;
- mit einem Behälter verbunden zu werden.

3. Katheter-Desobstruktionsvorrichtung (100) nach Anspruch 1 oder 2, wobei das Ablaufelement (114) eine allgemein T-förmige Ausgestaltung aufweist.

4. Katheter-Desobstruktionsvorrichtung (100) nach einem der Ansprüche 1 bis 3, wobei das Ablaufelement (114) und das flexible Katheterreinigungskabel (106) in einer Sterilverpackung (140) untergebracht sind.

5. Katheter-Desobstruktionsvorrichtung (100) nach einem der Ansprüche 1 bis 4, ferner umfassend eine Dichtung (112), die im Einsatz um das flexible KatheterReinigungskabel (106) angeordnet ist und das Strömen einer Flüssigkeit von der ersten Öffnung (126) zur zweiten Öffnung (128) blockiert.

6. Katheter-Desobstruktionsvorrichtung (100) nach Anspruch 5, wobei das Ablaufelement (114) eine erste Leitung (116), die sich von der ersten Öffnung (126) zu der zweiten Öffnung (128) erstreckt, und eine zweite Leitung (118), die von der zweiten Öffnung (128) abzweigt, umfasst, wobei die Dichtung (112) zwischen der zweiten Öffnung (128) und der Abzweigung der zweiten Leitung (118) angeordnet ist.

7. Katheterdesobstruktionssystem mit der Katheterdesobstruktionsvorrichtung (100) nach einem der Ansprüche 1 bis 6 und einem Katheterdesobstruktionsantrieb (200), wobei
der Katheter-Desobstruktionsantrieb (200) aufweist:
- eine Antriebskupplung (210), die dazu ausgebildet ist, mit der Kabelkupplung (110) des flexiblen Katheterreinigungskabels (106) verbunden zu werden;
- einen Motor (214), der die Antriebskupplung (210) rotatorisch antreibt;
- einen Aktor (216), der die Antriebskupplung (210) translatorisch bewegt;
- einen Kraftsensor (222), der dazu ausgebildet ist, die auf die Antriebskupplung (210) in der Translationsrichtung wirkende Kraft zu bestimmen; und
- eine Steuereinrichtung (202), die dazu ausgebildet, um den Motor (214) und den Aktor (216) zu steuern;
- wobei die Steuereinrichtung (202) zu Folgendem ausgebildet ist:
- den Motor (214) anzuweisen, die Antriebskupplung (210) zu drehen, und den Aktor (216) anzuweisen, die Antriebskupplung (210) in der proximalen Richtung zu bewegen.

8. Katheter-Desobstruktionssystem nach Anspruch 7, ferner umfassend:
- eine Öffnung (220), durch die das flexible Katheterreinigungskabel (106) geführt werden kann; und
- eine Dichtung (212), die an die Öffnung angrenzt, durch die das flexible Katheterreinigungskabel (106) geführt werden kann, und die verhindert, dass eine Flüssigkeit in den Katheter-Desobstruktionsantrieb (200) eintreten kann.

9. Katheter-Desobstruktionssystem (200) nach Anspruch 7 oder 8, wobei die Steuereinrichtung (202) ferner dazu ausgebildet ist:
- den Aktor (216) anzuweisen, die Antriebskupplung (210) um eine vorbestimmte Strecke in die proximale Richtung zu bewegen, und den Motor (214) anzuweisen, die Antriebskupplung (210) zu drehen;
- falls der Aktor (216) die Antriebskupplung (210) um die vorgegebene Strecke in der proximalen Richtung bewegt hat, den Aktor (216) anzuweisen, die Antriebskupplung (210) um eine vorgegebene Strecke in der distalen Richtung zu bewegen, und den Motor (214) anzuweisen, die Antriebskupplung (210) zu drehen; und
- falls der Aktor (216) die Antriebskupplung (210) um die zweite vorbestimmte Strecke in der distalen Richtung bewegt hat, den Aktor (216) anzuweisen, die Bewegung der Antriebskupplung (210) zu beenden, und den Motor (214) anzuweisen, die Drehung der Antriebskupplung (210) zu beenden.

10. Katheter-Desobstruktionssystem (200) nach Anspruch 9, wobei der Aktor (216) ein elektrischer Aktor ist und der Kraftsensor (222) ein Stromsensor ist, der den dem elektrischen Aktor zugeführten Strom misst.

## Revendications

1. Dispositif de désobstruction de cathéter (100), comprenant :
- un câble de nettoyage de cathéter flexible (106) qui comprend, au niveau de son extrémité proximale, un outil de désintégration (108) et au niveau de son extrémité distale, un couplage de câble (110), dans lequel le câble de nettoyage de cathéter flexible (106) est adapté de telle sorte qu'il soit introduit à l'intérieur d'une lumière d'un cathéter (30) ; et
- un élément de drain (114) qui comporte une première ouverture (126), une deuxième ouverture (128) et une troisième ouverture ; dans lequel :
- la première ouverture (126) est adaptée de telle sorte qu'elle soit connectée au cathéter (30) ;
- les première et deuxième (128) ouvertures sont adaptées de telle sorte que le câble de nettoyage de cathéter flexible (106) puisse passer au travers de la première ouverture (126) et de la deuxième ouverture (128) ;
- la troisième ouverture (113) est en communication en termes de fluide avec la première ouverture (126) ;
- la première ouverture (126) est opposée à la deuxième ouverture (128) ; et
- le câble de nettoyage de cathéter flexible (106) passe au travers de la première ouverture (126) et de la deuxième ouverture (128).

2. Dispositif de désobstruction de cathéter (100) selon la revendication 1, dans lequel la troisième ouverture (113) est adaptée de telle sorte qu'elle satisfasse au moins l'une des conditions qui suivent :
- elle soit connectée à un tube (122) ;
- elle soit connectée à un tuyau ;
- elle soit connectée à un sac (124) ; et
- elle soit connectée à un moyen de contenance.

3. Dispositif de désobstruction de cathéter (100) selon la revendication 1 ou 2, dans lequel l'élément de drain (114) présente une configuration en forme générale de T.

4. Dispositif de désobstruction de cathéter (100) selon l'une quelconque des revendications 1 à 3, dans lequel l'élément de drain (114) et le câble de nettoyage de cathéter flexible (106) sont localisés à l'intérieur d'un moyen de conditionnement stérile (140).

5. Dispositif de désobstruction de cathéter (100) selon l'une quelconque des revendications 1 à 4, comprenant en outre un moyen d'étanchéité (112) qui est agencé en utilisation autour du câble de nettoyage de cathéter flexible (106) de telle sorte qu'il bloque l'écoulement d'un liquide depuis la première ouverture (126) jusqu'à la deuxième ouverture (128).

6. Dispositif de désobstruction de cathéter (100) selon la revendication 5, dans lequel l'élément de drain (114) comprend un premier conduit (116) qui est étendu depuis la première ouverture (126) jusqu'à la deuxième ouverture (128) et un second conduit (118) qui est dérivé en termes de branchement à partir de la deuxième ouverture (128), dans lequel le moyen d'étanchéité (112) est agencé entre la deuxième ouverture (128) et la dérivation en termes de branchement du second conduit (118).

7. Système de désobstruction de cathéter comprenant le dispositif de désobstruction de cathéter (100) selon l'une quelconque des revendications 1 à 6 et un moyen d'entraînement et de commande de désobstruction de cathéter (200) ; dans lequel :
le moyen d'entraînement et de commande de désobstruction de cathéter (200) comprend :
- un couplage de moyen d'entraînement et de commande (210) qui est adapté de telle sorte qu'il soit connecté au couplage de câble (110) du câble de nettoyage de cathéter flexible (106) ;
- un moteur (214) qui entraîne en rotation le couplage de moyen d'entraînement et de commande (210) ;
- un moyen opératoire d'actionnement (216) qui déplace en translation le couplage de moyen d'entraînement et de commande (210) ;
- un capteur de force (222) qui est adapté de telle sorte qu'il détermine la force qui opère sur le couplage de moyen d'entraînement et de commande (210) dans la direction de translation ; et
- un contrôleur (202) qui est adapté de telle sorte qu'il commande le moteur (214) et le moyen opératoire d'actionnement (216) ; et dans lequel :
le contrôleur (202) est adapté de telle sorte qu'il réalise l'opération qui suit :
- il demande en instruction au moteur (214) d'entraîner en rotation le couplage de moyen d'entraînement et de commande (210) et le moyen opératoire d'actionnement (216) afin de déplacer le couplage de moyen d'entraînement et de commande (210) dans la direction proximale.

8. Système de désobstruction de cathéter selon la revendication 7, comprenant en outre :
- une ouverture (220) au travers de laquelle le câble de nettoyage de cathéter flexible (106) peut passer ; et
- un moyen d'étanchéité (212) qui est adjacent à l'ouverture au travers de laquelle le câble de nettoyage de cathéter flexible (106) peut passer et qui empêche qu'un liquide puisse pénétrer à l'intérieur du moyen d'entraînement et de commande de désobstruction de cathéter (200).

9. Système de désobstruction de cathéter (200) selon la revendication 7 ou 8, dans lequel le contrôleur (202) est en outre adapté de telle sorte qu'il réalise les opérations qui suivent :
- il demande en instruction au moyen opératoire d'actionnement (216) de déplacer le couplage de moyen d'entraînement et de commande (210) sur une distance prédéterminée dans la direction proximale et au moteur (214) d'entraîner en rotation le couplage de moyen d'entraînement et de commande (210) ;
- si le moyen opératoire d'actionnement (216) a déplacé le couplage de moyen d'entraînement et de commande (210) sur la distance prédéterminée dans la direction proximale, il demande en instruction au moyen opératoire d'actionnement (216) de déplacer le couplage de moyen d'entraînement et de commande (210) sur une seconde distance prédéterminée dans la direction distale et au moteur (214) d'entraîner en rotation le couplage de moyen d'entraînement et de commande (210) ; et
- si le moyen opératoire d'actionnement (216) a déplacé le couplage de moyen d'entraînement et de commande (210) sur la seconde distance prédéterminée dans la direction distale, il demande en instruction au moyen opératoire d'actionnement (216) d'arrêter de déplacer le couplage de moyen d'entraînement et de commande (210) et au moteur (214) d'arrêter d'entraîner en rotation le couplage de moyen d'entraînement et de commande (210).

10. Système de désobstruction de cathéter (200) selon la revendication 9, dans lequel le moyen opératoire d'actionnement (216) est un moyen opératoire d'actionnement électrique et le capteur de force (222) est un capteur de courant qui mesure le courant qui est alimenté sur le moyen opératoire d'actionnement électrique.
